# EUROPEAN PATENT SPECIFICATION

(11) **EP 0 847 376 B1**
(45) Date of publication and mention of the grant of the patent: **23.02.2005**
(21) Application number: 96928293.8
(22) Date of filing: 30.08.1996
(51) Int. Cl.: C04B 35/447, A61L 27/00, C04B 41/87

(54) **AN ARTIFICIAL STABILIZED COMPOSITION OF CALCIUM PHOSPHATE PHASES PARTICULARLY ADAPTED FOR SUPPORTING BONE CELL ACTIVITY**
ZUR UNTERSTÜTZUNG DER KNOCHENZELLENAKTIVITÄT BESONDERS GEEIGNETE STABILISIERTE ZUSAMMENSETZUNG AUS KALZIUMPHOSPHATPHASEN
COMPOSITION ARTIFICIELLE STABILISEE DE PHASES DE PHOSPHATE DE CALCIUM, PARTICULIEREMENT UTILE COMME SUPPORT DE CROISSANCE POUR DES CELLULES OSSEUSES

(30) Priority: 01.09.1995 US 3157 P; 21.12.1995 US 576238
(43) Date of publication of application: 17.06.1998
(73) Proprietor: MILLENIUM BIOLOGIX INC., Kingston, Ontario K7M 7G3 (CA)
(72) Inventor: PUGH, Sydney, M., Glenburnie, Ontario K0H 1S0 (CA); SMITH, Timothy, J., N., Kingston, Ontario K7M 1M1 (CA); SAYER, Michael, Kingston, Ontario K7M 1E4 (CA); LANGSTAFF, Sarah, Dorthea, Kingston, Ontario K7L 3W6 (CA)
(74) Representative: Lerwill, John
(86) International application number: PCT/CA1996/000585
(87) International publication number: WO 1997/009286

(56) References cited:
- WO-A-94/26872
- DATABASE WPI Week 8923, Derwent Publications Ltd., London, GB; AN 89-169871 & JP,A,1 111 763 (JGC CORP.) 28 April 1989
- DATABASE WPI Week 8446, Derwent Publications Ltd., London, GB; AN 84-284542 & JP,A,59 174 567 (AGENCY OF IND. SCI. TECH.) 3 October 1984
- ANATOMY AND EMBRYOLOGY, vol.170, no.3, December 1984 pages 247-247 - 256, XP002019145 S.J. JONES ET AL. cited in the application

## Description

This invention relates to bioactive artificial stabilized sintered compositions of calcium phosphate phases which are capable of supporting bone cell activity thereon. This invention has applications in medical diagnostics for the assessment of normal and abnormal bone cell activity as well as for medical therapeutics including bone and dental tissue replacement and repair as well as for *ex vivo* bone graft tissue engineering.

Bone is a complex mineralizing system composed of an inorganic or mineral phase, an organic matrix phase, and water. The inorganic mineral phase is composed of crystalline calcium phosphate salts while the organic matrix phase consists mostly of collagen and other noncollagenous proteins. Calcification of bone depends on the close association between the organic and inorganic phases to produce a mineralized tissue.

The process of bone growth is regulated to meet both structural and functional requirements. The cells involved in the processes of bone formation, maintenance, and resorption are osteoblasts, osteocytes, and osteoclasts. Osteoblasts synthesize the organic matrix, osteoid, of bone which after calcium phosphate crystal growth and collagen assembly becomes mineralized. Osteocytes regulate the flux of calcium and phosphate between the bone mineral and the extracellular fluid. Osteoclasts function to resorb bone and are essential in the process of bone remodelling. Disturbing the natural balance of bone formation and resorption leads to various bone disorders. Increased osteoclast activity has been demonstrated to lead to bone disease characterized by a decrease in bone density such as that seen in osteoporosis, osteitis fibrosa and in Paget's disease. All of these diseases are a result of increased bone resorption.

In order to understand the mechanisms involved which regulate bone cell functioning, it is important to be able to assess the normal function of bone cells and also the degree of perturbation of this activity in various bone diseases. This will lead to the identification of drugs targeted to restore abnormal bone cell activity back to within normal levels. Together with the identification of the etiology of abnormal and normal bone cell activity and the assessment of this activity, is the desire and need to develop compositions and methods for the treatment of abnormal bone cell activity, as a result of disease, surgical removal or physiological trauma all of which lead to bone tissue loss. Therapeutics which provide for the replacement and repair of bone tissue, such as with the use of bone implants, are highly desired.

Several research groups have developed methods to directly observe the activity of isolated osteoclasts *in vitro.* Osteoclasts, isolated from bone marrow cell populations, have been cultured on thin slices of natural materials such as sperm whale dentine (Boyde et al Brit. Dent. J. 156, 216, 1984) or bone (Chambers et al J. Cell Sci. 66, 383, 1984). The latter group have been able to show that this resorptive activity is not possessed by other cells of the mononuclear phagocyte series (Chambers & Horton, Calcif Tissue Int. 36, 556, 1984). More recent attempts to use other cell culture techniques to study osteoclast lineage have still had to rely on the use of cortical bone slices (Amano et al. and Kerby et al J. Bone & Min. Res. 7(3)) for which the quantitation of resorptive activity relies upon either two dimensional analysis of resorption pit areas of variable depth or stereo mapping of the resorption volume. Such techniques provide at best an accuracy of approximately 50% when assessing resorption of relatively thick substrata. In addition these analysis techniques are also very time consuming and require highly specialized equipment and training. Furthermore, the preparation and subsequent examination of bone or dentine slices is neither an easy nor practical method for the assessment of osteoclast activity.

The use of artificial calcium phosphate preparations as substrata for osteoclast cultures has also met with little success. Jones et al (Anat. Embryol 170, 247, 1984) reported that osteoclasts resorb synthetic apatites *in vitro* but failed to provide experimental evidence to support this observation. Shimizu et al (Bone and Mineral 6, 261, 1989) have reported that isolated osteoclasts resorb only devitalized bone surfaces and not synthetic calcium hydroxyapatite. These results would indicate that functional osteoclasts are difficult to culture *in vitro.*

Several groups have also attempted to provide compositions suitable for the therapeutic replacement of bone tissue. US Patent No. 4,871,578 discloses a process for the formation of a non-porous smooth coating of hydroxyapatite suitable for implant use. US Patent No. 4,983,182 discloses a ceramic implant which comprises a sintered body of zirconia and a coating of α-TCP and zirconia, or hydroxyapatite and zirconia. US Patent No. 4,988,362 discloses a composition for the fusion of a bioceramic to another bioceramic. US Patent No. 4,990,163 discloses a coating used for the production of bioceramics which consist of α-TCP and β-TCP. Although these different compositions may be used as biocompatible coatings for implants and the like, none of these compositions have been demonstrated to be suitable for the culture of both active osteoclasts and osteoblasts in a reliable and reproducible manner such to allow for the quantitative assessment of the specific activity of osteoclast resorption and osteoblast secretion of bone matrix. Furthermore, none of the prior compositions developed, can be manipulated to reliably produce a range of films, thicker coatings and bulk ceramic pieces which share a common composition and morphology which leads to similar bioactive performance *in vivo* and *in vitro.*

Applicant's published international PCT patent application WO94/26872 describes a sintering process for forming thin films of calcium phosphate phases on which bone cell function occurs. This is the first thin layer of synthetic material on which osteoclasts can exhibit extended activity and on which osteoblasts may secrete bone matrix. As described in that application, a variety of factors should be considered in providing a thin film with a desired ratio of hydroxyapatite to tricalcium phosphate. Such parameters include:
1) amounts of reagents for preparing the sol-gel hydroxyapatite substance;
2) rate of combination of reagents;
3) duration and rate of mixing when making the sol- gel;
4) rates and methods of precipitation and separation;
5) process environmental conditions during the manufacture of the sol-gel;
6) velocity of removal of the substrate from the sol-gel in dip coating a film thereon;
7) sintering temperature;
8) sintering in a controlled atmosphere such inert gas, a vacuum or an atmosphere with water vapour present.
9) the nature of the substrate, with quartz being a preferred embodiment to create a transparent substrate coated with stabilized calcium phosphate phases.

It was suggested in this earlier PCT patent application, that in order to obtain a broad range in ratios of hydroxyapatite to tricalcium phosphate on quartz substrates, many of these parameters needed to be considered in order to achieve the ratios of 10:90 through to 90: 10. The suggested sintering temperatures in an air atmosphere were from approximately 800°C to approximately 1100°C. It was established that at 800°C the film was predominantly hydroxyapatite. A sintering temperature of about 900°C provided ratios of about 70:30. At 1000°C, the ratio was about 10:90 and at 1100°C the film was predominantly tricalcium phosphate. It was also suggested that sintering in a vacuum at 1000°C produced a ratio of approximately 66:34. It has now been found that the preferred ratios are from 50:50 to 20:80. The optimum ratio is approximately 333:666. To achieve these ratios; consideration can be given to several of the above factors. However, it is desirable to minimize the variability in several of the above factors and to achieve the desired ratios for optimum film compositions in an exacting reproducible manner. Surprisingly, this film is stable in the presence of various aqueous media, even though α-tricalcium phosphate is supposed to be soluble in water.

Applicants have discovered that the presence of stabilizing entities can stabilize the composition and prevent its degradation in physiological fluids. Hence, disappearance of calcium phosphate entities from a film, coating or bulk ceramic piece of this composition, is substantially due to the activity of the osteoclasts and not due to a dissolution process. The stabilized artificial bioactive composition is the first such composition which supports both osteoclast and osteoblast activity and which allows for the reliable assessment of the physiological activities of both cell types as well as for the development of both diagnostic and therapeutic strategies. The stabilizing entities stabilize the α-tricalcium phosphate within the calcium phosphate phases formed during sintering to provide for a stable form of α-tricalcium phosphate which does not degrade in physiological fluids and which forms calcium phosphate phases having a cell compatible morphology which supports and encourages bone cell activity thereon.

The present invention provides a stabilized composition which provides a wide variety of diagnostic and therapeutic applications. The stabilized composition, in accordance with an aspect of the invention, can be used to provide a range of thin films, coatings, powders and bulk ceramic pieces which share a common surface globular microporisity and an internal microporosity. In addition, the bulk ceramics also can have a macroporosity within the structure in order to provide an artificial three dimensional bone tissue similar to that found *in vivo.* The composition, made in any form, encourages the activity of bone cells cultured thereon and also allows for the development of *ex viva* engineered artificial bone tissues to use as bone grafts.

In accordance with one aspect of the invention, there is provided a bioactive artificial sintered composition for consistently supporting bone cell activity, said composition comprising; stabilized tricalcium phophate stabilized entities selected from the group consisting of silicon entities, aluminium entities, zirconium entities, barium entites, titanium entities, germanium entities, chromium entities, vanadium entities, niobium entities, boron entities and mixtures thereof, wherein said stabilized tricalcium phosphate is insoluble in physiological fluids of pH of approximately 6.4 to 7.3 said composition being obtainable by the conversion, by sintering, of a hydroxyapatite sol-gel substance uniformly doped with said stabilizing entities.

In one embodiment, said composition is obtainable by the conversion of a hydroxyapatite sol-gel substance uniformly doped with a metal-organic solution of said stabilizing entities by sintering.

According to another aspect of the invention, there is provided a process for making a stabilized artificial sintered composition of calcium phosphate phases having a morphology suitable for supporting bone cell activity thereon, said process comprising the steps of: doping a hydroxyapatite substance with stabilizing entities selected from the group consisting of silicon entities, aluminium entities, zirconium entities, barium entities, titanium entities, germanium entities, chromium entities, vanadium entities, niobium entities, boron entities and mixtures thereof; sintering said doped hydroxyapatite substance, wherein sintering converts said doped hydroxyapatite substance into primarily alpha tricalcium phosphate within phosphate phases, wherein said stabilized alpha tricalcium phosphate is insoluble in physiological fluids of pH of about 6.4 to 7.3, is resorbable by osteoclasts and promotes secretion of mineralizing collagenous matrix by osteoblasts, the method being characterized in that said hydroxyapatite substance is in the form of a sol-gel, and in that said hydroxyapatite sol-gel is uniformly doped with said stabilizing entities.

A bioactive sintered artificial microporous polycrystalline structure for consistently supporting bone cell activity thereon is also described herein the structure comprising sintered substantially uniformly stabilized calcium phosphate phases having a globular surface morphology of loosely interconnected rounded granules with interconnected micropores in the structure wherein said substantially uniformly stabilized calcium phosphate phases are developed by the conversion of a hydroxyapatite substance, substantially uniformly doped with stabilizing entities, at sintering temperatures into tricalcium phosphate, wherein said substantially uniformly stabilized alpha tricalcium phosphate is insoluble in physiological fluids and wherein said stabilizing entities are added to the hydroxyapatite prior to sintering.
Figure 1 is a predominance area diagram showing the effect of CaO activity on the stabilities of hydroxyapatite and tricalcium phosphate.
Figure 2 is a plot showing the phases of calcium phosphate entities formed in the presence of stabilizing silicon entities from the conversion of the hydroxyapatite substance of the present invention.
Figure 3 is a plot showing the effect of CaO/Al₂O₃ on the activity of CaO.
Figure 4 is a plot showing the effect of CaO/TiO₂ and CaO/B₂O₃ ratios on the activity of CaO.
Figure 5 comprises graphs (a), (b) and (c) which show results of energy dispersive x-ray spectroscopy (a) at the interface of the composition with the substrate; (b) just above the interface and (c) at the top of a film produced by diffusion of silica entities from the substrate.
Figure 6 is a cross-sectional SEM of mineralized collagenous bone matrix deposited on a stabilized thin film composition of the present invention by active osteoblasts.
Figure 7 (a) is a photograph showing the deposition of florescent calcified bone matrix produced by osteoblasts cultured on the stabilized composition.
Figure 7 (b) is a photograph showing a control in which no osteoblasts are cultured on the stabilized thin film composition and no fluorescent calcified bone matrix is visualized.
Figure 8 is a SEM of osteoclast resorption pits on bulk three dimensional solid ceramics composed of artificial bioactive stabilized compositions.
Figure 9 is a SEM of osteoclast resorption pits on thin films of artificial bioactive stabilized compositions.
Figure 10 is a cross-sectional TEM magnified micrograph of a thin layer of the artificial stabilized composition on a quartz substrate showing the morphology.
Figure 11 is a SEM micrograph of the stabilized composition applied as a thin film showing the surface microporous structure.
Figure 12(a)is a SEM micrograph of a commercial sintered hydroxyapatite in the absence of stabilizing entities.
Figure 12(b) is a SEM micrograph of commercial sintered hydroxyapatite in the presence of silicon stabilizing entities.
Figure 13 is a SEM of an osteoclast on natural bone showing a natural resorption pit.
Figure 14 is a SEM micrograph illustrating the globular surface morphology of a bulk ceramic made by the process of the present invention.

The composition of calcium phosphate substance is that provided in accordance with that described in the applicant's published PCT application WO94/26872. This process provides on a consistent basis, a thin film of calcium phosphate phases which are within the desired range of 50:50 to 20:80 for the ratio of hydroxyapatite to α-tricalcium phosphate. It has now been found that the presence of stabilizing entities significantly and unexpectantly stabilizes the α-TCP within the calcium phosphate phases to provide a bioactive composition which supports and encourages the activity of both osteoblasts and osteoclasts and which allows for the quantification of such activity in a reproducible manner, and provides for the development of diagnostic and therapeutic strategies for bone tissue loss.

It is to be understood that the term "stabilized" refers to the calcium phosphate phases formed upon conversion of the hydroxyapatite which maintain a consistent crystallographic and chemical structure when placed in ambient conditions or in a physiological environment *in vivo* or *in vitro.* It is also to be understood that the term "bioactive" refers to the ability to support osteoblastic bone growth over and throughout structures substantially or exclusively made of the present composition and simultaneously promote natural controlled extracellular resorption of the composition by osteoclasts, while avoiding non-specific chemical and/or cellular dissolution and/or degradation, in a process closely resembling that of normal bone turnover. Such bioactivity being present during *in vitro* and *in vivo* uses of the materials where bone cells are present. By the term "calcium phosphate phases", it is intended to include the various calcium phosphate species in the sintered product such as hydroxyapatite α-TCP, β-TCP, calcium octophosphate, tetracalcium phosphate and dicalcium phosphate.

It was initially thought that for supporting bone cell activity *in vitro,* the suitable calcium phosphate product was to be pure or essentially pure hydroxyapatite and was understood to be the calcium phosphate entity of choice in making a film. It has now been determined that materials which are predominantly of hydroxyapatite do not encourage normal function of osteoclasts and osteoblasts and, in actual fact, in the presence of osteoclasts, very little activity can be observed. It was found, however, that by providing a mixture of calcium phosphate phases which include hydroxyapatite and α-tricalcium phosphate, the degree of resorption is encouraged through a broad range where the film predominantly of α-tricalcium phosphate provides the highest degree of resorption, whereas a film predominantly of hydroxyapatite provides a negligible degree of resorption. It is this realization, with respect to the presence of α-tricalcium phosphate that partially suggests why the presently developed calcium phosphate materials encourage functional properties in bone cells being cultured on such materials. This aspect, in providing stabilized calcium phosphate phases in the form of a thin film which permits, for example, transmittance of light or light reflection, allows one to carry out diagnostic procedures to evaluate several functional properties of bone cells being cultured on such films.

Surprisingly, it has been found that standardizing the preparation of the hydroxyapatite sol-gel substance and selecting a very specific range of sintering temperatures, not only achieves the desired ratios but also reveals that the optimum composition is formed by conversion of hydroxyapatite as prepared by the sol-gel process to α-tricalcium phosphate. Little or no β-tricalcium phosphate has been detected in these preferred optimized film compositions. There is no need to prepare mixtures of hydroxyapatite and α-tricalcium phosphate preparations of the individual phases. Instead, the technique as described in published PCT application WO 94/26872 is sufficient in preparing a sol-gel hydroxyapatite substance. The chemical reaction for making such a hydroxyapatite substance in a medium of elevated pH is as follows: ${\text{5Ca(NO}}_{\text{3}} {\text{)}}_{\text{2}} {\text{+ 3NH}}_{\text{4}} {\text{H}}_{\text{2}} {\text{PO}}_{\text{4}} {\text{+ 7NH}}_{\text{4}} \text{OH} {\text{→ Ca}}_{\text{5}} {\text{(PO}}_{\text{4}} {\text{)}}_{\text{3}} {\text{OH + 10NH}}_{\text{4}} {\text{NO}}_{\text{3}} {\text{+ 6H}}_{\text{2}} \text{O}$

The starting solutions consist of aqueous solutions in which the reactants are fully dissolved and which can be well mixed. The hydroxyapatite forms as fine particles in suspension, the size of which is shown by light scattering experiments to grow from an average of about 0.3 µm to over 1 µm when aging the sol-gel substance for 24 hours after preparation.

The hydroxyapatite substance is stable in neutral and/or alkaline media. Preferably the reaction medium is brought to an elevated pH usually in the range of about 12. A first phosphate solution is added drop by drop into a second calcium solution to prevent the formation of tetracalcium monohydrogen triphosphate thereby obtaining a product of the desired hydroxyapatite. The sol-gel substance can be filtered, dried as a powder, calcined and fired in an alumina crucible at 1000°C, to form calcium hydroxyapatite phases which are stable under normal conditions of atmospheric humidity. The conversion of this phase at sintering temperatures of over 1200°C is into primarily α-tricalcium phosphate with smaller amounts of some other phases also being formed such as β-TCP, calcium octophosphate, tetracalcium phosphate or dicalcium phosphate. It is to be understood by those skilled in the art other "contaminating" materials may also be formed in the sintered stabilized calcium phosphate phases. Such materials may also be added to the hydroxyapatite substance before sintering. The presence or addition of such contaminants preferably does not affect the composition and the morphology of the stabilized composition in any manner which will affect the support of bone cell activity thereon.

With respect to the sintering process, it has also been found that sintering of the dried film of hydroxyapatite substance may be carried out in a standard type of high temperature oven without any need to control the atmosphere in the oven. When a new oven is used or an oven contaminated by previous use for other purposes, it is preferred to cycle the oven through the sintering temperature range several times while the oven is empty. Such preconditioning of the oven removes any volatiles and prepares it for use. No additional steps are required. Ambient air may be present in the oven during the break-in period and during normal use for sintering coated substrates where the presence of ambient air does not hamper the process and results in producing consistent results for the desired ratio. Under these conditions, the sintering temperature may range from 920°C up to 1100°C in providing the desired ratios of 50:50 up to 20:80 in the presence of a quartz substrate. It has been found that as the temperature increases, the conversion of hydroxyapatite into α-tricalcium phosphate is also increased. At sintering temperatures in the range of 920°C up to 950°C the ratio may vary from 50:50 towards 333:666. At selected sintering temperatures in the range of 950°C to 1000° the ratio is approximately 333:666. Increasing the temperature beyond a 1000°C and up to 1100°C further increases the conversion and produces compositions having ratios in the range of 333:666 to 20:80. The preferred sintering temperature is approximately 975°C where the ratio of 333:666 is achieved.

The conversion of the hydroxyapatite to tricalcium phosphate occurs via the reaction; ${\text{2Ca}}_{\text{5}} {\text{(OH)(PO}}_{\text{4}} {\text{)}}_{\text{3}} {\text{→ 3Ca}}_{\text{3}} {\text{(PO}}_{\text{4}} {\text{)}}_{\text{2}} {\text{+ CaO + H}}_{\text{2}} \text{O}$the degree of conversion at any temperature being sensitive to the partial pressure of water in the surrounding atmosphere and to factors which modify the concentration of CaO.

The nature of the tricalcium phosphate which is formed is of significance. For non-stoichiometric hydroxyapatite with a Ca/P ratio of 1.5 - 1.60 (Nakamura, Thermochimica Acta, Vol. 165, 1990), and for many commercially supplied hydroxyapatite powders (Aldrich Chem Co.), β-tricalcium phosphate is often formed when the powder is heated to 1100°C and then cooled to temperatures below 1000°C. β-TCP is a stable, insoluble compound which appears in nature as the mineral Whitlockite. In the conversion of the sol-gel derived hydroxyapatite substance formed from aqueous solutions as described herein, and in calcium hydroxyapatite powders formed from alternate precipitation reactions, it is found that the formation of α-tricalcium phosphate is enhanced at temperatures below 1000°C in the presence of stabilizing entities. In the development of calcium phosphate based coatings, α-TCP has not been a great subject of attention because of its degradation in physiological fluids due to its relatively high solubility and from the fact that it only results from the high temperature conversion of pure hydroxyapatite at temperatures of over 1250°C.

From the conversion equation, it is expected that any factor which controls the activity of CaO in the system will modify both the temperature and the reversibility of the hydroxyapatite conversion. The addition of a stabilizing entity such as SiO₂ is believed to react with CaO by the reaction: ${\text{CaO + SiO}}_{\text{2}} {\text{→ CaSiO}}_{\text{3}}$thereby driving the conversion to lower temperatures. This reaction should be complete for 1 mol SiO₂ per 1 mol CaO produced in the reaction. Other reactions to form different silicates with other CaO/SiO₂ ratios may be possible.

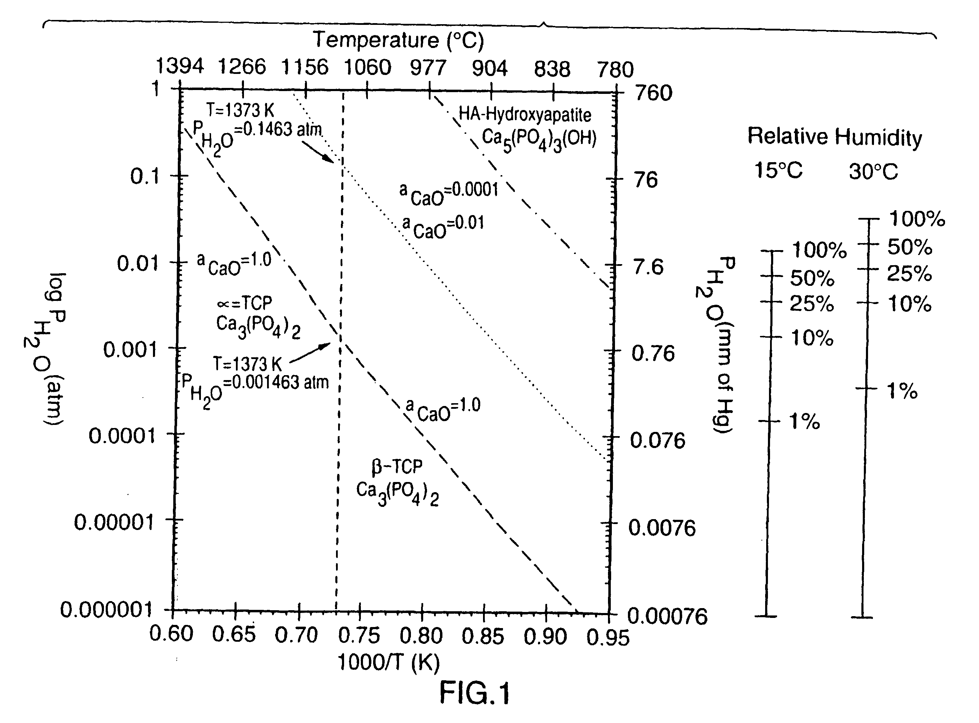

When CaO is removed by the action of the silica to form calcium silicates, the temperature at which the TCP phases form is reduced to temperatures consistent with the data from the conversion of the prepared hydroxyapatite composition as seen in Figure 1. The addition of silicon entities drives the conversion line to the right, that is, to lower temperatures with the formation of primarily α-TCP.

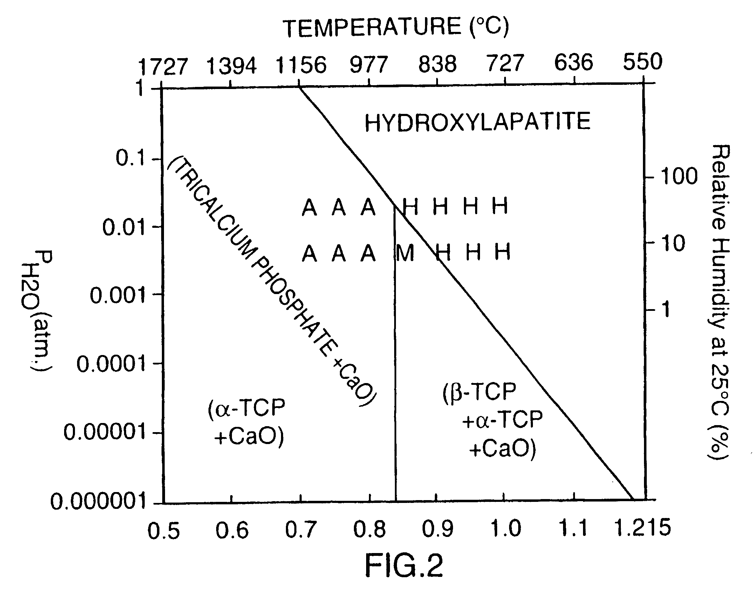

The proposed mechanism by which silica plays a direct role in encouraging the formation of the alpha tricalcium phosphate compared to other phases such as beta tricalcium phosphate is that the silicon entities enter the hydroxyapatite crystal structure and stabilizes the alpha phase with respect to beta. It has now also been demonstrated, in accordance with a preferred embodiment, that the nature of the starting hydroxyapatite substance and the manner in which silica is added is of importance. When silica in the form of a powder is added to a commercial pure hydroxyapatite powder, and co-milled to promote mixing, the conversion product observed at high sintering temperatures of over 1000°C was β-TCP. In contrast, powders prepared according to the present invention with silica added as a metal-organic solution, converted to primarily a stabilized alpha tricalcium phosphate phase which was retained at low temperature as shown in Figure 2 at the 950°C line. This conversion is not reversible. At high temperature, the doped powders show a reduction in the conversion temperature from over 1200°C for pure powders to about 950°C for silica doped powders. As noted, this development is believed to be due to the formation of calcium silicates, whereby the stabilized resulting phase composition is retained on cooling to low temperature.

One reason why powders with stabilizing entities prepared in accordance with the present invention have reproducible and stable phase compositions with a desired surface morphology and an internal microporous structure is that the hydroxyapatite substance is originally prepared in the sol-gel process as very fine particles. Addition of stabilizing entities such as silicon entities in the form of a metallorganic solution allows each of these particles to be in intimate contact with a layer of silicon entities resulting in thorough mixing. On sintering, the silica is in close proximity to the CaO released in the conversion reaction. It is proposed that the formation of insoluble calcium silicate entities at the surface of each particle limits the reversibility of the reaction and plays a role in preventing the solubility of alpha tricalcium phosphate in aqueous physiological media.

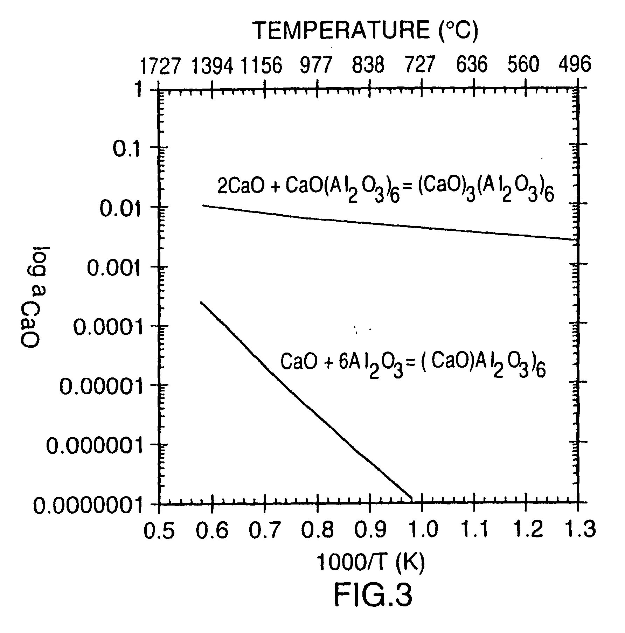

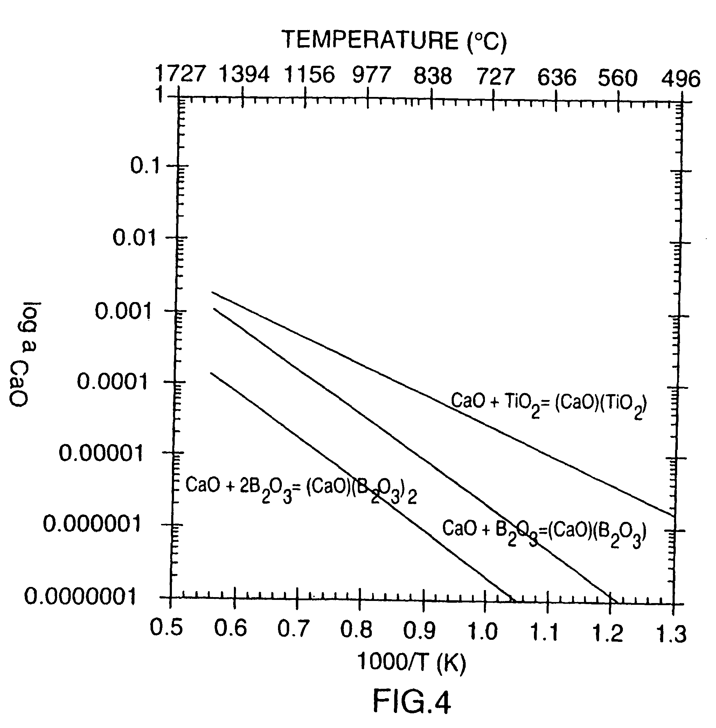

In a similar manner to silica, titanium, aluminum and boron were predicted to reduce the conversion temperature and thus can be used as stabilizers, ie. dopants. Figures 3 and 4 illustrate the temperature reduction with the formation of CaO/Al/Ti/Ba complexes. These metals may be used to remove the CaO from the hydroxyapatite and result in a stabilized α-TCP. The factors important for the selection of stabilizer (dopant) and of the compound by which it is dispersed are: (a) it needs to interact with formed CaO which forms a stable calcium compound, (b) it must be capable of being dispersed uniformly throughout the sol-gel substance preferably in a manner which surrounds the outer surfaces of the newly formed particles, (c) it should not stabilize undesirable phases within the calcium phosphate system, and (d) it must be non-toxic when integrated for biological applications. Stabilizing entities suitable for use in the present invention are those which form oxides, preferably metal oxides. The metal oxides are those selected which produce the desired composition and morphology and are selected from the group consisting of aluminum, zirconium, germanium, chromium, vanadium and niobium, and are more preferably selected from silicon and titanium oxides. Mixtures of such stabilizing entities may also prove useful.

Sintering is done in the presence of stabilizing entities. The stabilizing entities are provided by virtue of the addition of stabilizing entities to the hydroxyapatite substance before sintering. The stabilizing entities are provided in an amount sufficient to stabilize the calcium phosphate phases which are in the form of thin films, powders, thicker coatings, bulk ceramic pieces and in bulk ceramic pieces having an internal macroporosity formed therein. Preferably for the support and encouragement of bone cell activity, it is the resultant unique bioactive surface morphology and internal microporous structure which are reproducible and which are a function of the presence of the stabilizing entities during the sintering process.

Depending on its proposed use, the composition can be provided as various structures such as in the form of thin films for diagnostics or as thicker coatings to be used on bone or dental implants. Herein, thin films can be described as those having a thickness of 0.1 microns to 5 microns, thicker coatings are those having a thickness above 5 microns designed to be applied to other substrates. Bulk ceramic pieces refer to larger three-dimensional structures which are functionally independent of a substrate, by sintering a film of hydroxyapatite substance on the face of a quartz substrate.

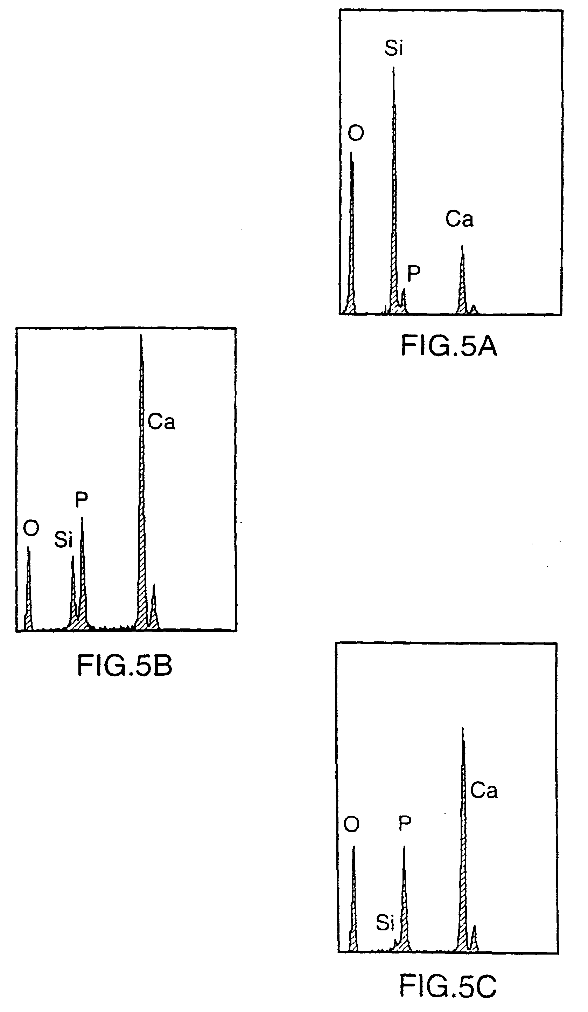

By sintering a film of hydroxyapatite substance on the face of a quartz substrate, the quartz substrate provides a sufficient source of silicon entities which can diffuse throughout the calcium phosphate phases and produce a sufficient silicon entity content. As shown in Figure 5 (a), the interface of the composition with the substrate, Figure 5 (b) just above the interface, and Figure 5 (c) of the top of the film, however, the silicon entities are not uniformly distributed throughout the film composition. During the sintering period, silicon entities are released from the surface of the quartz and diffuse through the surface of the hydroxyapatite substance layer.

The stabilizing entities of the present invention are selected from metal and non-metal oxides of silicon, aluminum, zirconium, boron, titanium, germanium, chromium, vanadium, niobium and mixtures thereof. Substrates containing or made of aluminum, zirconium, boron, titanium and various mixtures of these components may be suitable for providing the source of stabilizing entities.

The thin film as provided on a suitable support, in accordance with this invention, significantly advances the study and understanding of bone cell functional properties. The make-up of the stabilized film, as provided in accordance with this invention, permits the culture of various types of bone cells thereon. The surface make-up may be adjusted to encourage a significant degree of resorption of the calcium phosphate entities of the film material through to a negligible degree of resorption of the calcium phosphate entities in the study of osteoclast activity. Similarly, osteoblast activity may be studied by detecting a build up of calcified bone matrix. The ability to provide the material in a film which is sufficiently thin that resorption of the entities by osteoclasts can be detected by the disappearance of resorbed calcium phosphate entities provides a simple inexpensive format for analysis compared to the prior art techniques. The film make-up as made in accordance with this invention, supports the biological function of bone cells. The benefit in providing the film on a transparent supporting substrate, such as quartz or glass, lends to easy evaluation techniques of the diagnostic process including automated machine reading.

Ideally the film thickness is greater than 0.1 micron because it has been found that at film thicknesses less than 0.1 microns it is difficult to obtain uniform film coverage, free from discrete voids. As to the upper thickness limit for the film, it can be of any desired thickness depending upon its end use. As will be discussed, the degree of resorption may be detected by light transmittance, which preferably requires a film less than 10 microns in thickness. The substrate is of quartz which readily withstands the required sintering temperatures and has the desired degree of transparency to permit light transmittance tests to determine the extent of resorption of calcium phosphate entities from the film material.

The developed thin films may be used in kits and the like to provide for assessment of bone cell activity. The film may be embodied in the form of a "kit" comprising quartz substrates, pre-coated with an adherent calcium phosphate thin film, which may be used in a cell culture vessel (possibly a 24-well optionally sterilized multi-well plate i.e. of approximately 15 mm diameter) as a system suitable for the culture of mixed bone cell populations. The device is simple and relies on only routine laboratory equipment and techniques for use, is suitable for quantitative analysis, and is inexpensive to fabricate but strong enough to withstand normal levels of handling and may be packaged in lots, of (for example) 24 samples in a plastic presentation box. The thin film surfaces have a defined and reproducible chemistry and are mechanically strong enough to withstand transport when used with an appropriate packing material.

In each case the culture conditions may be such that osteoclasts, in either mononuclear or multinucleate form could be expected to survive in a functional state and resorb the artificial calcium phosphate of the film. Similarly, osteoblast are also capable of actively secreting calcified bone matrix under such culture conditions.

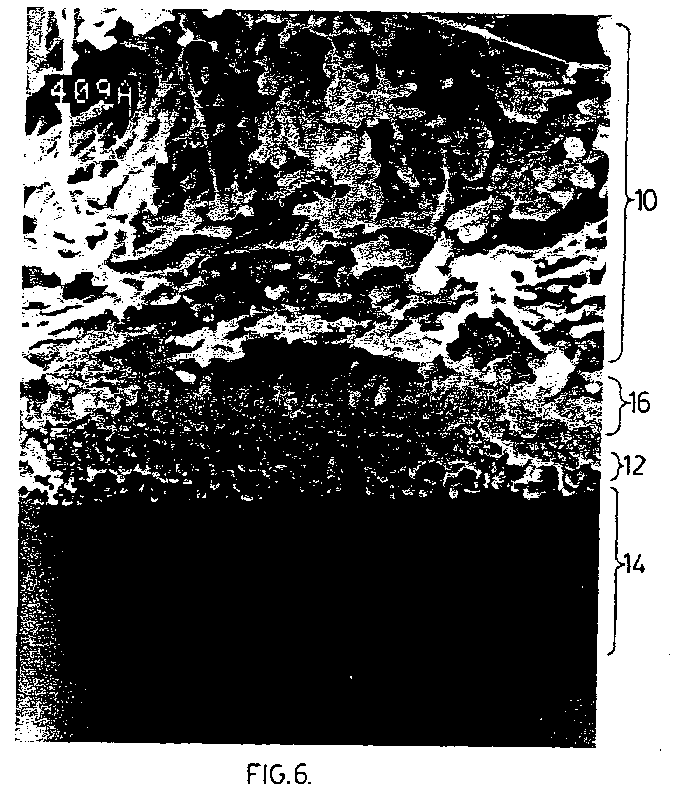

These substrates may be used to assess the resorptive activity of osteoclasts and monitor the change in this level of resorptive activity either as a result of a disease process or the inclusion, in the culture medium, of an agent such as a drug which would influence, either directly or indirectly, osteoclastic resorptive activity. The substrates are also suitable for the culture of active osteoblasts in order to observe and assess the secretion of bone matrix thereon as well as use the deposited mineralized matrix for *in vivo* transplantation. As seen in Figure 6, mineralized collagenous matrix 10 is deposited by cultured osteoblasts on the surface of the stabilized thin film 12 as provided on a quartz substrate 14. A well integrated boundary layer 16 resembling a cement line is shown and which is similar to the same type of cement lines formed by osteoblasts *in vivo* at the interface between new bone and old bone. This clearly suggests that the pressure stabilized composition allows for physiological osteoblast activity further supporting the role of the stabilized composition as an important bone remodelling product.

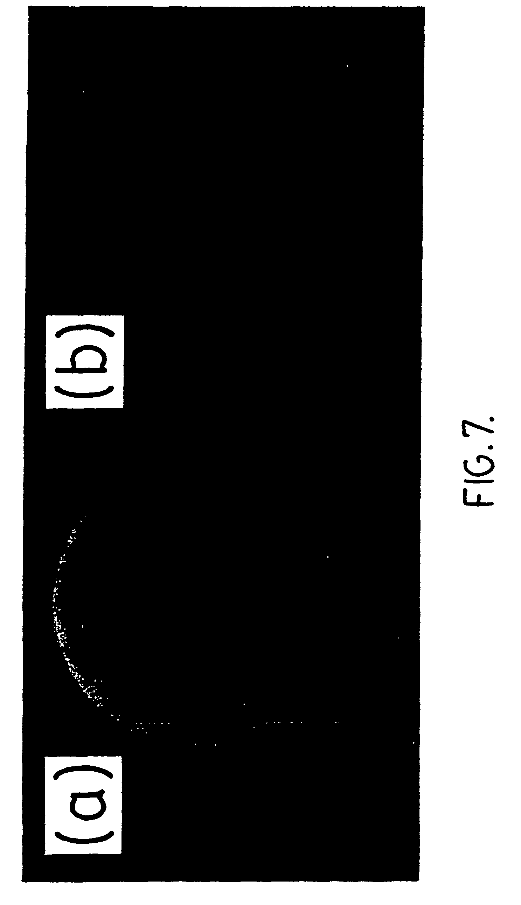

The device may be used as a means of quantifying the resorptive activity of osteoclasts or build-up of bone-like material by the activity of osteoblasts. Such activity analysis may occur under continuous real-time monitoring, time-lapse intervals or end-point determination. The steps in establishing bone cell activity are common to each of the above monitoring schedules in that bone cells (either animal or human) are cultured, in specific conditions, on one or more of the devices. The culture period is from several hours to many days and preferably from approximately 2 to 10 days (the optimum time is cell species and protocol dependent), during which time the extent of osteoclast activity may be continuously monitored, periodically monitored, or simply not monitored on an on-going basis in favour of final-end-point determination. Similarly, osteoblast activity may be observed by determining extent of calcified bone matrix build-up. As is shown in Figure 7, a quartz disc coated with a stabilized film of the present invention and simultaneously cultured with osteoblasts (a), is highly fluorescent indicating the presence of mineralized bone matrix. In contrast, a stabilized film coated on quartz in the presence of medium alone (b) shows no fluorescence. The amount of calcified bone matrix is directly proportional to the measurable fluorescence emitted. Tetracycline is a naturally fluorescent material. As the cells take up tetracycline, it is metabolized and its metabolites are secreted and incorporated into the newly formed bone matrix. The tetracyline will only fluoresce upon its being metabolized by the osteoblasts. This demonstrates that osteoblasts actively secrete bone matrix on the stabilized composition.

Once the sol-gel hydroxyapatite substance is prepared, it may be applied as a thin film to the desired substrate in a variety of techniques. For example, the dip-coating method (C.J. Brinker et al., Fundamentals of Sol-Gel Dip Coating, Thin Solid Films, Vol. 201, No. 1, 97-108, 1991) consists of a series of processes: withdrawal of the substrate from a sol or solution at a constant speed, drying the coated liquid film at a suitable temperature, and firing the film to a final ceramic.

In spin-coating the sol-gel is dropped on a plate which is rotating at a speed sufficient to distribute the solution uniformly by centrifugal action. Subsequent treatments are the same as those of dip coating.

It is appreciated that there are a variety of other techniques which may be used to apply a thin film of the sol-gel to the substrate. Other techniques include a spraying of the sol-gel, roller application of the sol-gel, spreading of the sol-gel and painting of the sol-gel.

An alternative to coating discrete discs of a singular size is to coat an enlarged substrate with a film of the sol-gel. The entire film on the substrate is then sintered. A device, such as a grid, may then be applied over the film to divide it into a plurality of discrete test zones.

In these various techniques of the sol-gel substance application, the thickness and quality (porosity, microstructure, crystalline state and uniformity) of formed films are affected by many factors. These include the physical properties, composition and concentration of the starting sol, the cleanliness of the substrate surface, withdrawal speed of the substrate and the firing temperature. In general the thickness depends mainly on the withdrawal rate and sol viscosity for a dip coating process. Since heterogeneity in the sol is responsible for the formation of macropores and cracks, the coating operation should be undertaken in a clean room to avoid particulate contamination of the sol. At the heat-treatment stage, high temperatures are required to develop the required microstructure and desired conversion of hydroxyapatite to α-tricalcium phosphate.

The purpose of applying the dip coating method to fabricate calcium phosphate films is twofold: (a) to make films with required qualities (uniformity, thickness, porosity, etc); and (b) to make translucent calcium phosphate films on transparent substrates for biological experiments.

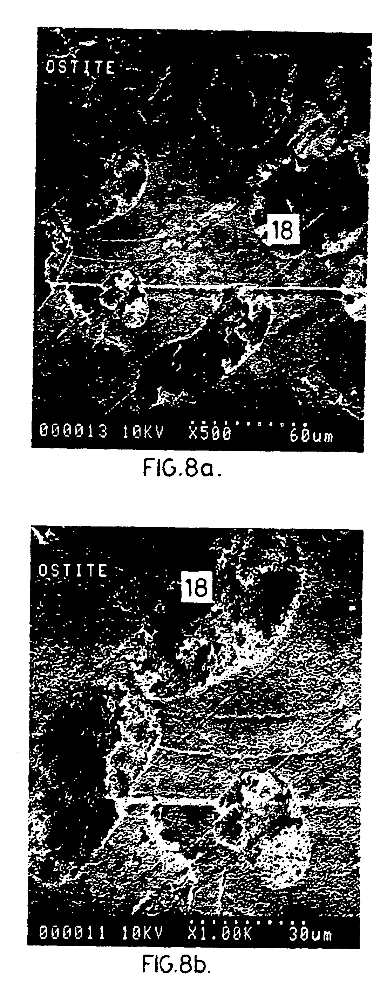

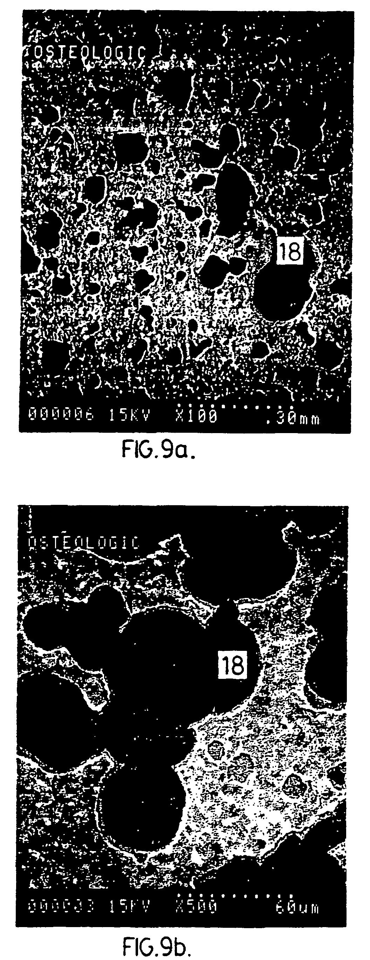

It has also been found that the stabilized artificial composition of the present invention is suitable for the production of not only thin films and thicker coatings, but also powders and bulk ceramics. Ceramics are prepared from sintered powders prepared by the sol-gel method described herein with the addition of silica to create a desired stabilized hydroxyapatite/alpha tricalcium phosphate phase mixture. In one embodiment the sintered powder is finely ground in an amount sufficient to produce a disc 0.5 - 1mm thick, which is then mixed with a drop of retained sol-gel substance of the same dopant composition to create a damp powder which helps in keeping the particles together. The damp powder is uniaxillally pressed in a laboratory die at a pressure of about 5 tonnes/cm². The resultant bulk material shows good green strength and is fired at 1000°C for 1 hour in air. Such ceramics retain all of the same characteristics as the stabilized composition as used as a thin film, or coating. For SiO₂ stabilized compositions, X-ray diffraction showed little change in the phase composition between the initial powder and the final ceramics. The surface topography as seen in Figure 8, in plan view, is strikingly similar to that of the composition coated on a quartz substrate illustrated in Figure 9. The resorptive capability of the osteoclasts on thin films and the bulk ceramics are very similar. Osteoclast resorption is observed as the presence of resorption pits 18 on the bulk ceramic, and are similar to resorption pits 18 witnessed on the thin films (Figures 8 and 9).

As recognised by one skilled in the art, larger ceramic bulk pieces could be formed by shaping of the ceramic to form bulk pieces for use in desired applications. The bulk pieces produced retain the desired stabilized calcium phosphate phase composition as well as the microporous globular surface morphology, and internal microporous structure both of which facilitate bone cell activity thereon.

A particular aspect of ceramic preparation for use in biological applications is the fabrication of ceramic pieces with a fine globular surface microporosity and internal microporosity which leads to bioactivity, and a larger macrostructure of pores of dimensions 50-1000 µm or more, within the internal structure. This encourages bone remodelling in a system more closely resembling physiological *in vivo* bone remodelling. Such macroporosity at the low end of the range being particularly suited to applications desiring rapid ingrowth of bone matrix, while macroporosity at the high end of the range allow cells to access the interior for uses such as for *ex vivo* tissue engineering production of bone grafts. Using powders doped with a stabilizing entities such as silica and sintered prior to use, porous ceramics can be made by mixing of such powders with styrene balls of a desired size. After pressing a dampened doped powder with styrene balls, then at a required pressure, the styrene is then removed by pyrolysis at temperatures of about 400°C - 600°C. The porous ceramic is then fired to 1000°C in the normal manner as previously described. This procedure results in the formation of a bulk ceramic having an external globular microporous structure, an underlying internal microporous structure and an internal macroporous structure allowing cells to migrate and function throughout the entire bulk ceramic unit.

It is to be understood by those skilled in the art that materials similar to styrene may be used to develop macroporosity within the ceramic structure. Other materials which are capable of pyrolysis at temperatures below the normal sintering temperatures are also useful to form the macroporous structure. The materials used should also not leave any toxic residues. It is also understood that other methods can also be used to farm the macrostructure such as mechanical drilling of holes, the use of lasers or use of foaming agents.

Since the significant aspect of the present invention is the mixture of hydroxyapatite and alpha tricalcium phosphate phases coupled with the surface morphology created by doped powder formed from the sol-gel hydroxyapatite substance, one skilled in the art will understand that other methods of fabricating films, coatings and bulk structures from this powder may also be used in accordance with the present invention. This includes the use of known techniques such as plasma or thermal spray or electrophoretic deposition.

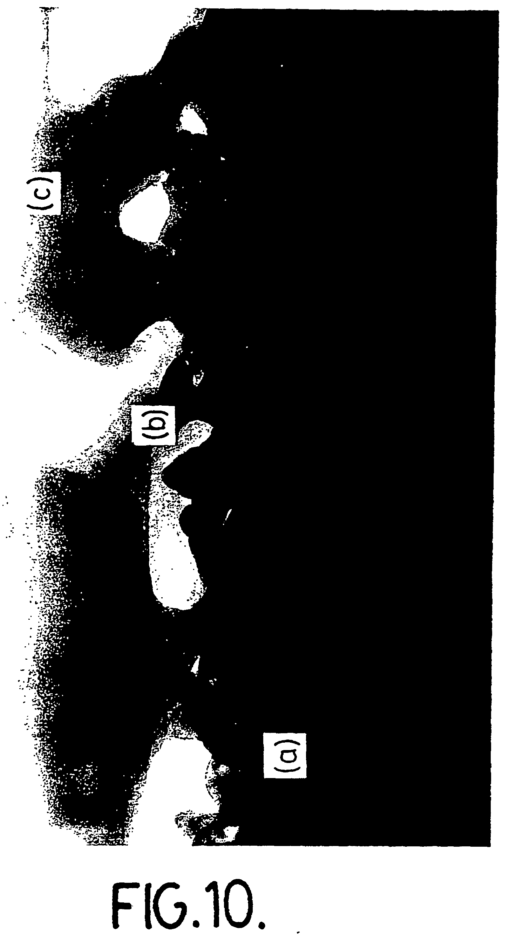

With reference to Figure 10, a cross-sectional TEM micrograph shows a gradient of layers in the form of the quartz substrate (a), the interface layer (b), which comprises small grains, and (c) the upper layer which includes the surface of the film which is comprised of small crystallites embedded in granules which provides the globular microporous structure. During the sintering process, silicon entities are released from the quartz (a) and which diffuse through the hydroxyapatite as it is converted to α-tricalcium phosphate in forming the sintered thin layer. The interface layer (b) has a smaller crystalline structure than the surface which has larger polycrystalline granules of the calcium phosphate phases.

The morphology of the artificial sintered composition of the present invention is unique and has not been previously reported or demonstrated. We have now discovered a surface morphology presenting a loosely interconnected globular structure of rounded granules having a microporous structure of interconnected pores. In accordance with a preferred aspect of this invention, the morphology successfully supports cultures of functional osteoclasts and osteoblasts.

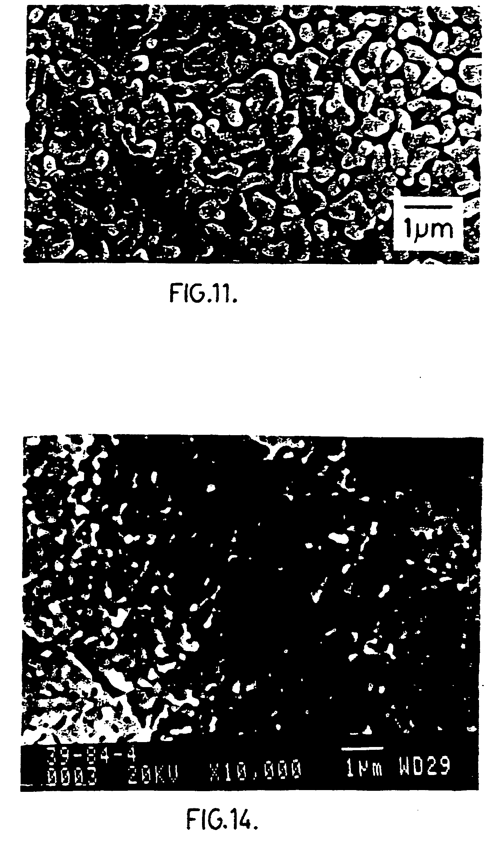

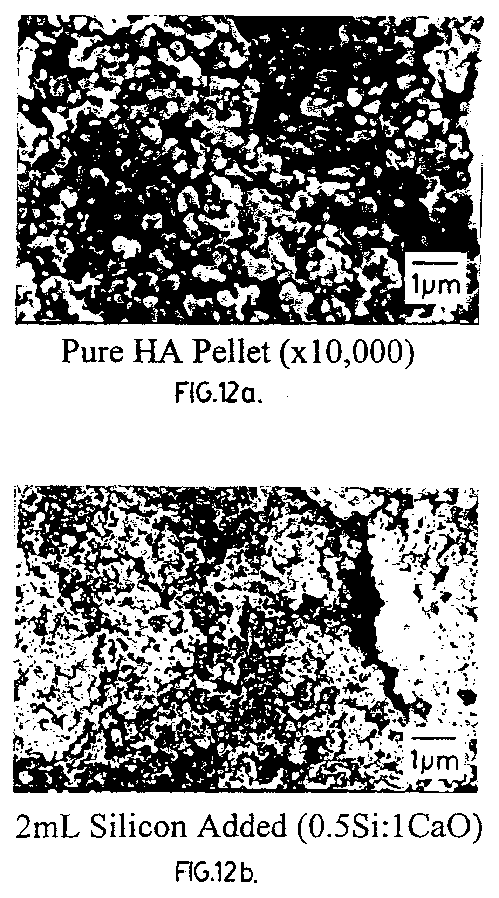

The surface morphology of the coating has a characteristic form involving a loosely interconnected globular structure resembling coral (Figure 11). The size of the granules varies from approximately 0.5-1µm in lateral dimension. The coating is porous in the direction perpendicular to the substrate . with a larger planar density closer to the surface than near to the substrate. This morphology may allow for the percolation of liquid media and other physiological fluids within the coating. In contrast, the surface morphology of hydroxyapatite prepared from other coprecipitation methods, does not result in a microporous structure as provided by the present invention. As seen in Figures 12(a) and 12(b), the surface morphology of such prepared hydroxyapatite films in the absence (a) and presence (b) of stabilizing entities is not microporous as compared to the present composition seen in Figure 11. In addition, is has been reported that synthetic polycrystalline hydroxyapatite is not resorbed by osteoclasts (Shimizu, Bone and Minerology, Vol. 6, 1989).

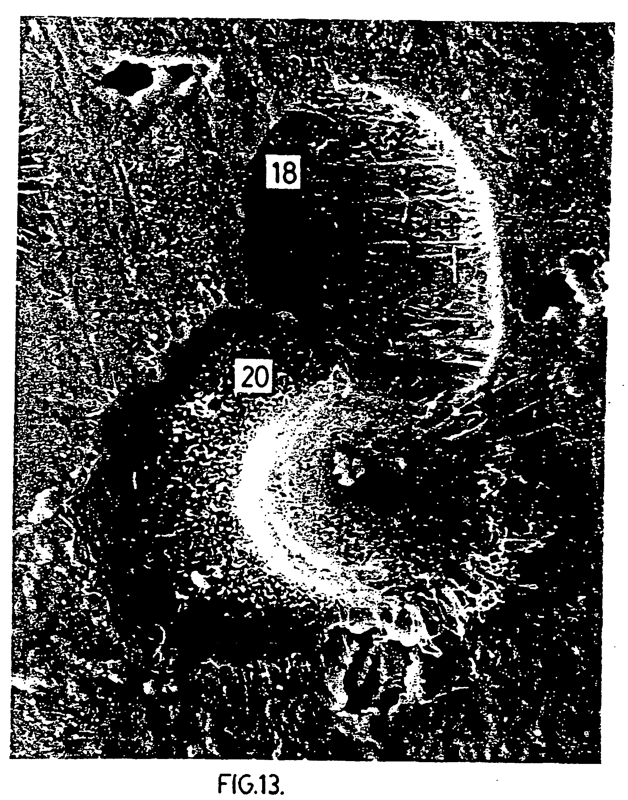

The globular surface morphology is made up of rounded granules comparable in size to the aggregated deposits initially made by an osteoblast cell in the process which leads to bone formation. The present composition provides a surface morphology compatible with the type of morphology the cell expects to encounter *in vivo.* A typical osteoclast resorption pit is shown in Figure 13, in which the substrate is bone. As demonstrated in Figure 9, the resorption pits 18 formed by osteoclasts 20 on the present artificial composition are extremely similar to those seen on natural bone of Figure 13 which suggests that osteoclasts function similarly in both systems. This implies that the surface morphology of the artificial sintered composition is compatible with the type of morphology the cell expects to encounter *in vivo.*

The bulk microporosity of the stabilized composition may ensure that the calcium or phosphate ion concentrations near the surface of the artificial material are within the limits expected by the cell as encounter *in vivo* with natural bone which is made up of hydroxyapatite, collagen and other fibrous tissues. During osteoclast mediated extracellular dissolution processes which lead to resorption, this complex material leads to a particular local concentration of dissolution products. During the dissolution or resorption of fully inorganic artificial hydroxyapatite or α-TCP, the resulting concentration limits for certain cellular behaviours are narrowly defined, for the cell to achieve an activity on artificial surfaces comparable to that on natural bone, means must be available to adjust the local concentration levels of elements such as calcium. The porosity of the composition allows this to occur through flow or diffusion of media.

The stabilized bioactive artificial composition of the present invention provides a unique chemical composition together with a unique surface morphology and internal microporous structure that has never previously been demonstrated. Compositions have not been previously reported which demonstrate consistent bone cell bioactivity *in vivo* and *in vitro* and which *in vitro* can be readily, accurately and repetitively quantified. The nature of the stabilized composition is versatile in that it can be provided in a powder, thin film, thick coating, bulk ceramic piece or macroporous bulk ceramic piece. In each case, the unique surface morphology and internal microporosity is maintained as well as the stabilized calcium phosphate phase composition. As seen in Figure 14, the microporous surface morphology is maintained on a bulk ceramic prepared from the present stabilized composition.

The stabilized composition of the present invention is ideal for *in vitro* diagnostics in order to characterize abnormal bone cell functioning in a large scale and automated manner. The stabilized artificial composition is also readily applicable as a coating for bone and dental implants to promote tissue regeneration and repair. The structure of the composition is such that is it very similar and therefore compatible with bone tissues and cells *in vivo* which will avoid the problems of rejection of foreign materials.

The present composition has the required physical characteristics and affinity/compatibility with *in vivo* hard tissue such that it can be used for a wide variety of therapeutic applications such as for providing implants *in vivo* as well as for the regeneration and repair of bone tissue *in vivo* as for example in hip and knee replacements, fractures, and dental implants. The composition can also be used for various tissue engineering applications performed *ex vivo* in order to provide artificially produced bone material which can then be transplanted as bone grafts *in vivo* for bone tissue replacement, regeneration and repair. Patients can provide the supply of osteoclasts and osteoblasts used to culture on the composition in order to lessen the chances of tissue rejection thereby producing fully compatible bone grafts. Alternatively, donor bone cells may also be used for this purpose. Such grafts can be prepared for tissue replacement in the absence or presence of the bone cells used to produce the bone tissue. It is preferred, however, that cell-containing grafts be from autologous donors in order to minimize problems associated with tissue rejection. The stabilized composition, in powder form, may also be used for medical therapeutics. Stabilized powders may be mixed and suspended within polymeric substances, compatible with tissues and non-toxic, and then applied *in vivo* for the filing of voids within bone tissues.

All of the applications in which the present composition can be used have the advantage that both osteoclasts and osteoblasts function actively with the composition in any form thus providing for a bone tissue system much like that found *in vivo.* The artificial bioactive composition of the present invention promotes both osteoconduction and resorption so that normal tissue healing and regeneration can occur while simultaneously allowing the artificial material to be resorbed in the process of normal bone tissue remodelling.

The following procedures exemplify aspects of the invention for providing a bioactive artificial sintered composition which has stabilized calcium phosphate entities therein and which also displays a unique morphology capable of supporting bone cell activity thereon.

### Procedure 1, Preparation of Hydroxyapatite Sol-Gel Substance

The following procedure is based on preparing sufficient sol-gel hydroxyapatite for manufacturing purposes. Solution A comprises a calcium nitrate tetrahydrate and Solution B comprises an ammonium dihydrogen orthophosphate (mono basic). Solution A is mixed with Solution B to produce the desired sol-gel, Solution C. Solution A is prepared by adding 40 mls of doubly distilled water to 4.722 grams of calcium nitrate, Ca(NO₃)₂. The solution is stirred at moderate speed for sufficient time to dissolve all of the calcium nitrate which is normally in the range of 3 minutes. To this solution, 3 mls of ammonia hydroxide (NH₄OH) is added and stirred for approximately another 3 minutes. The pH of the solution is tested where a pH of about 12 is desired. To this solution is added 37 mls of double distilled water to provide a total solution volume of approximately 80 mls. The solution is stirred for another 7 minutes and covered.

Solution B is prepared by adding 60 mls of double distilled water to a 250 ml beaker containing 1.382 grams of NH₄H₂PO₄. The beaker is covered and stirred at moderate speed for 3 to 4 minutes until all NH₄H₂PO₄ is dissolved. To this solution is added 71 mls of NH₄OH and the beaker then covered and stirring continued for approximately another 7 minutes. The pH of the solution is tested where a pH of about 12 is desired. To this is added another 61 mls of double distilled water and the beaker covered to provide a total solution volume of approximately 192 mls. The solution is then stirred for a further 7 minutes and covered.

The desired sol-gel is then prepared by combining Solution B with Solution A. All of Solution A is introduced to a 500 ml reagent bottle. Stirring is commenced at a moderate speed and Solution B introduced to the reagent bottle at a rate of approximately 256 mls per hour until all 192 ml of Solution B is delivered into Solution A. An excess of Solution B may be used to compensate for any solution which may remain in the 250 ml beaker or any tubing used in the transfer process. After completion of this addition and combination of Solution A with Solution B, the resultant Solution is continued to be stirred at moderate speed for approximately 23 to 24 hours. The resultant sol-gel is inspected for any abnormal precipitation or agglomeration. If any abnormal precipitation or agglomeration has occurred, the solution must be discarded and preparation commenced again. The sol is then carefully transferred to another 500 ml reagent bottle so as to avoid any inclusion of particle agglomerations that may be present on the walls of the original reagent bottle. Approximately 240 mls of Solution C, that is the resultant sol-gel, is delivered to a centrifuge bottle and centrifuged for 20 minutes at about 500 rpm at room temperature. Following centrifugation, 180 mls of supernatant is discarded without disturbing the sediments. The sediments are gently resuspended by mixing in a smooth rotating manner for about 30 minutes. Viscosity of the sol-gel is then measured and preferably is between 20 to 60 cP. The sol-gel is then ready for dip coating of the selected substrate or for other applications.

### Procedure 2 - Preparation of Silica Doped Hydroxyapatite Substance

A silica solution is prepared as follows. The amounts determined create approximately 0.168 g SiO₂/4ml solution. 4 mls of silica solution is added to 60 ml of centrifuged hydroxyapatite sol-gel substance created in procedure 1 and reacted with 0.168 g CaO produced in the conversion reaction.

| Silicon Solution Components | |
|---|---|
| Tetrapropyl orthosilicate Si(OC₃H₇)₄ | 7.32 gm |
| 2-methoxyethanol CH₃OCH₂CH₂OH | 34.5 gm |

The silica solution is added to the hydroxyapatite substance prepared by procedure 1, such that the concentration of SiO₂ is of the ratio 1M of SiO₂/l mol CaO produced upon conversion during sintering.

### Procedure 3 - Preparation of Thin Film Formats

Before the application of the thin film to the substrate, the substrate needs to be thoroughly cleaned to ensure satisfactory film coverage. In the case of quartz substrates, cleaning is achieved by placing the discs in a glass beaker and supplying chromic acid cleaning solution to the glass beaker to cover all discs. The beaker is then covered. The discs are then sonicated in a water bath for 1 hour. The acid is washed away using tap water for 20 minutes. The residual tap water is removed by three changes of doubly distilled water. After the final change of double distilled water, every single disc is dried with lint-free towel and inspected for flaws in the quartz surface. Any residual particulate on the surface is removed as needed with compressed nitrogen or air. The discs are stored in covered trays in an aseptic environment. This method can be used to clean any type of quartz substrate.

The quartz disc substrate, or other substrate having an appropriate composition, is dipped in the doped sol-gel prepared by Procedure 2. The disc is grasped at the edges to avoid touching the surface. The disc is dipped in the sol, preferably by machine. The disc is removed from the sol at a prescribed withdrawing velocity. The coating on one side of the disc is removed. The coated substrate is then placed in a clean Petrie dish and covered and dried at room temperature. The film, as formed prior to sintering, should be uniform without cracks, clumps or voids. It is understood that the dip coating process as applied to a face of a disc, may also be applied to any other shape of substrate, such as, a flat rectangular shaped substrate of quartz.

### Procedure 4 - Preparation of Dry Hydroxyapatite Powder

The sol-gel substance prepared by procedure 2 is dried at 100°C for approximately 8 hours. The dried substance is then ground using a mortar and pestle or any other mechanism which can grind and produce a fine powder. The powder can then be sintered following the standard sintering process, as described in Procedure 7, except that the powder is sintered in a crucible and then again ground after cooling. The same procedure can be followed for the preparation of a doped or stabilized hydroxyapatite.

### Procedure 5 - Production of Bulk Ceramic Pieces

Ceramics (three-dimensional bulk pieces) have been created from silica doped hydroxyapatite powders according to the following. A doped sol-gel substance was produced according to procedure 2. Part of the sol-gel substance was saved and the remainder filtered. The powder was dried at 120°C and ground to produce a fine powder. About 0.09 gm of powder was placed in a plastic dish. A glass dropper was used to form a drop of the initial sol-gel substance, which weighed about 0.055 gm when mixed with the powder. The sol was mixed with the powder to form a damp, but not wet paste. The damp paste was packed into a stainless steel die of diameter 6.25 mm and pressed at 2 metric tonnes for 1 minute. The bulk piece was removed from the press, air dried, and fired in a covered alumina crucible according to Procedure 7. Surface morphology was very similar to that of the artificial sintered thin films as seen in Figure 14.

### Procedure 6 - Preparation of Bulk Ceramic Pieces having a Macrostructure of Pores

Hydroxyapatite prepared powders which are doped with a stabilizing entity such as silica as in Procedure 2 and sintered at 1000°C, are mixed with styrene balls of a desired size, dampening the powder with additional sol-gel substance, and pressing at a pressure of about 1 tonne/cm² so as not to extrude the styrene. Enhanced green strength is achieved in such pressed powder/styrene compacts using a damping mixture of retained sol and 2.5 wt % solution of polyvinyl alcohol. The styrene is removed by pyrolysis by heating in air or oxygen to 550°C. The macroporous ceramic is then fired to 1000°C in the manner as described for normal sintering in Prodecure 7.

### Procedure 7 - Sintering of the Hydroxyapatite Substance

The following sintering process may be carried out in standard laboratory furnaces of various sizes, capable of operating at temperatures from ambient up to at least 1100°C, and designed to maintain accurate and stable internal temperatures, particularly between 800°C and 1100°C, such as Lindberg models 51744 or 894-Blue M. The components prepared by Procedures 3, 4, 5 or 6 are carefully transferred onto a standard ceramic plate (as is common practice in the Lindberg oven). The ceramic plate is used as a carrier during the sintering process to facilitate easy loading and withdrawal of multiple substrates from the furnace. The furnace temperature is set to the temperature required to achieve the desired ratios of HA:α-TCP. Utilizing a programmable furnace such as the Lindberg model 894-Blue M, the furnace may be programmed to hold the desired temperature, which will normally be selected from the range 920°C to 1100°C, for a maximum of one hour to ensure desired diffusion of the silicon entities through the developed gradient layers of hydroxyapatite and α-tricalcium phosphate. In the case of non-programmable furnaces, a separate timer may be used to warn the operator to turn the furnace off at the end of the required sintering time at selected temperature. The ceramic plate carrying the sintered substrates is removed at any time after the internal furnace temperature has cooled to an acceptable and safe touch-temperature of approximately 60°C. Individual substrates may then be stored or packaged for final use.

In accordance with this process, thin films and thicker coatings of hydroxyapatite/α-tricalcium phosphate can be produced on a consistent basis having the desired composition where variability in the various processing parameters have been minimized to ensure such consistency.

Although preferred embodiments of the invention are described herein in detail, it will be understood by those skilled in the art that variations may be made thereto without departing from the scope of appended claims.

## Claims

1. A bioactive artificial sintered composition for consistently supporting bone cell activity, said composition comprising;
- stabilized tricalcium phosphate stabilized by stabilizing entities selected from the group consisting of silicon entities, aluminium entities, Zirconium entities, barium entities, titanium entities; germanium entities, chromium entities, vanadium entities, niobium entities, boron entities and mixtures thereof, wherein said stabilized tricalcium phosphate is insoluble in physiological fluids of pH of approximately 6.4 to 7.3, **characterised in that** said composition is obtainable by the conversion, by sintering, of a hydroxyapatite sol-gel substance uniformly doped with said stabilizing entities.

2. A composition according to claim 1, wherein sintering is carried out at a temperature of between 900°C and 1100°C.

3. A composition according to claim 1 or 2, wherein said stabilized tricalcium phosphate is primarily alpha tricalcium phosphate.

4. A composition according to claim 1, 2 or 3, wherein said composition is in the form of a powder, coating or a three-dimensional bulk material.

5. A composition according to claims 1, 2, 3 or 4, wherein said stabilising entities are provided in the form of a metal-organic solution.

6. A composition according to any preceding claim, wherein said stabilising entities are silicon entities.

7. A composition according to claim 6, wherein said silicon entities are tetrapropyl orthosilicate.

8. A process for making a stabilized artificial sintered composition of calcium phosphate phases having a morphology suitable for supporting bone cell activity thereon, said process comprising the steps of:
- doping a hydroxyapatite substance with stabilizing entities selected from the group consisting of silicon entities, aluminium entities, zirconium entities, barium entities, titanium entities, germanium entities, chromium entities, vanadium entities, niobium entities, boron entities and mixtures thereof;
- sintering said doped hydroxyapatite substance, wherein sintering converts said doped hydroxyapatite substance into primarily alpha tricalcium phosphate within phosphate phases, wherein said stabilized alpha tricalcium phosphate is insoluble in physiological fluids of pH of about 6.4 to 7.3, is resorbable by osteoclasts and promotes secretion of mineralizing collagenous matrix by osteoblasts, the process being **characterised in that** said hydroxyapatite substance is in the form of a sol-gel, and **in that** said hydroxyapatite sol-gel is uniformly doped with said stabilising entities.

9. A process according to claim 8, wherein said stabilising entites are in the form of a metal-organic solution.

10. A process according to claim 9, wherein the doping step comprises dispersing said stabilising entities throughout said sol-gel in a manner which surrounds the outer surfaces of said phosphate phases.

11. A process according to claim 8, 9 or 10, wherein the hydroxyapatite sol-gel is made by the mixing of a calcium nitrate tetrahydrate solution and an ammonium dihydrogen orthophosphate solution.

12. A process according to claim 8, 9, 10 or 11 wherein the composition formed is a powder, coating or a three-dimensional bulk material.

13. A process according to claim 8, 9, 10, 11 or 12 wherein said silicon entites are tetrapropyl orthosilicate.

14. A process according to claim 13, wherein said tetrapropyl orthosilicate is in solution with 2-methoxyethanol.

15. A process according to any one of claims 8 to 14, wherein said calcium phosphate phases are in a ratio of 50:50 to 20:80 for the ratio of hydroxyapatite to alpha tricalcium phosphate.

16. A process according to any one of claims 8 to 15, wherein sintering of the hydroxyapatite substance is done at temperatures of about 900°C to 1100°C.

17. A sintered artificial microporous polycrystalline structure for supporting bone cell activity, said structure being made by the process of any one of claims 8 to 15.

18. A polycrystalline structure according to claim 17, wherein said structure is an implant.

19. An *in vitro* method for the culturing of functional bone cells, said method comprising applying a suspension of bone cells in physiological media to a composition according to any one of the claims 1 to 7 provided on a substrate.

## Patentansprüche

1. Bioaktive künstliche, gesinterte Zusammensetzung zur beständigen Unterstützung der Knochenzellaktivität, wobei genannte Zusammensetzung Folgendes umfasst:
- stabilisiertes Tricalciumphosphat, das durch stabilisierende Entitäten stabilisiert ist, die aus der Gruppe ausgewählt sind, bestehend aus Silicium-Entitäten, Aluminium-Entitäten, Zirconium-Entitäten, Barium-Entitäten, Titan-Entitäten, Germanium-Entitäten, Chrom-Entitäten, Vanadium-Entitäten, Niobium-Entitäten, Bor-Entitäten und Gemischen davon, worin genanntes stabilisiertes Tricalciumphosphat in physiologischen Flüssigkeiten mit einem pH von ca. 6,4 bis 7,3 unlöslich ist, **dadurch gekennzeichnet, dass** genannte Zusammensetzung durch die Umwandlung mittels Sintern einer mit genannten stabilisierenden Entitäten gleichmäßig dotierten Hydroxyapatit-Sol-Gel-Substanz erhaltbar ist.

2. Zusammensetzung nach Anspruch 1, worin das Sintern bei einer Temperatur zwischen 900 °C und 1100 °C durchgeführt wird.

3. Zusammensetzung nach Anspruch 1 oder 2, worin genanntes stabilisiertes Tricalciumphosphat hauptsächlich α-Tricalciumphosphat darstellt.

4. Zusammensetzung nach Anspruch 1, 2 oder 3, worin genannte Zusammensetzung in der Form eines Pulvers, einer Beschichtung oder eines dreidimensionalen Bulkmaterials vorliegt.

5. Zusammensetzung nach Anspruch 1, 2, 3 oder 4, worin die genannten stabilisierenden Entitäten in der Form einer metallorganischen Lösung bereitgestellt sind.

6. Zusammensetzung nach einem der vorangehenden Ansprüche, worin die genannten stabilisierenden Entitäten Silicium-Entitäten darstellen.

7. Zusammensetzung nach Anspruch 6, worin die genannten Silicium-Entitäten Tetrapropylorthosilicat darstellen.

8. Verfahren zur Herstellung einer stabilisierten, künstlichen, gesinterten Zusammensetzung aus Calciumphosphatphasen mit einer Morphologie, die zur Unterstützung der Knochenzellaktivität darauf geeignet ist, wobei das Verfahren die folgenden Schritte umfasst:
- Dotieren einer Hydroxyapatit-Substanz mit stabilisierenden Entitäten, die aus der Gruppe ausgewählt sind, bestehend aus Silicium-Entitäten, Aluminium-Entitäten, Zirconium-Entitäten, Barium-Entitäten, Titan-Entitäten, Germanium-Entitäten, Chrom-Entitäten, Vanadium-Entitäten, Niobium-Entitäten, Bor-Entitäten und Gemischen davon;
- Sintern genannter dotierter Hydroxyapatit-Substanz, worin das Sintern die genannte dotierte Hydroxyapatit-Substanz in hauptsächlich α-Tricalciumphosphat in Phosphatphasen umwandelt, worin genanntes stabilisiertes α-Tricalciumphosphat in physiologischen Flüssigkeiten mit einem pH von ca. 6,4 bis 7,3 unlöslich ist, durch Osteoklasten resorbierbar ist und die Sekretion von mineralisierender Kollagenmatrix durch Osteoblasten fördert, wobei das Verfahren **dadurch gekennzeichnet ist, dass** genannte Hydroxyapatit-Substanz in der Form eines Sol-Gels vorliegt und dass genanntes Hydroxyapatit-Sol-Gel mit genannten stabilisierenden Entitäten gleichmäßig dotiert ist.

9. Verfahren nach Anspruch 8, worin genannte stabilisierende Entitäten in der Form einer metallorganischen Lösung vorliegen.

10. Verfahren nach Anspruch 9, worin der Dotierschritt das Dispergieren genannter stabilisierender Entitäten durch genanntes Sol-Gel hindurch auf eine Weise umfasst, die die Außenflächen von genannten Phosphatphasen umgibt.

11. Verfahren nach Anspruch 8, 9 oder 10, worin das Hydroxyapatit-Sol-Gel durch das Mischen einer Calciumnitrattetrahydrat-Lösung und einer Ammoniumdihydrogenorthophosphat-Lösung hergestellt wird.

12. Verfahren nach Anspruch 8, 9, 10 oder 11, worin die gebildete Zusammensetzung ein Pulver, eine Beschichtung oder ein dreidimensionales Bulkmaterial darstellt.

13. Verfahren nach Anspruch 8, 9, 10, 11 oder 12, worin genannte Silicium-Entitäten Tetrapropylorthosilikat darstellen.

14. Verfahren nach Anspruch 13, worin sich genanntes Tetrapropylorthosilicat in Lösung mit 2-Methoxyethanol befindet.

15. Verfahren nach einem der Ansprüche 8 bis 14, worin genannte Calciumphosphatphasen in einem Verhältnis von 50 : 50 bis 20 : 80 für das Verhältnis von Hydroxyapatit zu α-Tricalciumphosphat vorliegen.

16. Verfahren nach einem der Ansprüche 8 bis 15, worin das Sintern der Hydroxyapatit-Substanz bei Temperaturen von ca. 900 °C bis 1100 °C durchgeführt wird.

17. Gesinterte, künstliche, mikroporöse, polykristalline Struktur zur Unterstützung der Knochenzellaktivität, wobei genannte Struktur mithilfe des Verfahrens nach einem der Ansprüche 8 bis 15 hergestellt wird.

18. Polykristalline Struktur nach Anspruch 17, worin genannte Struktur ein Implantat darstellt.

19. *In-vitro*-Verfahren zum Kultivieren funktionsfähiger Knochenzellen, wobei genanntes Verfahren das Applizieren einer Suspension aus Knochenzellen in physiologischen Medien auf eine Zusammensetzung nach einem der Ansprüche 1 bis 7, die auf einem Substrat bereitgestellt ist, umfasst.

## Revendications

1. Composition frittée artificielle bioactive en vue du soutien cohérent de l'activité cellulaire osseuse, ladite composition comprenant :
- du phosphate de tricalcium stabilisé en stabilisant des entités sélectionnées parmi le groupe constitué des entités de silicone, des entités d'aluminium, des entités de zirconium, des entités de baryum, des entités de titane, des entités de germanium, des entités de chrome, des entités de vanadium, des entités de niobium, des entités de bore et de mélanges de celles-ci, dans laquelle ledit phosphate de tricalcium stabilisé est insoluble dans les fluides physiologiques d'un pH d'environ 6,4 à 7,3, **caractérisée en ce que** ladite composition peut être obtenue par la conversion, par frittage, d'une substance sol-gel d'hydroxyapatite additionnée uniformément desdites entités stabilisantes.

2. Composition selon la revendication 1, dans laquelle le frittage est réalisé à une température comprise entre environ 900°C et 1100°C.

3. Composition selon la revendication 1 ou 2, dans laquelle ledit phosphate de tricalcium stabilisé est principalement du phosphate d'alpha-tricalcium.

4. Composition selon la revendication 1, 2 ou 3, dans laquelle ladite composition est sous la forme d'une poudre, d'un revêtement ou d'un matériau brut tridimensionnel.

5. Composition selon la revendication 1, 2, 3 ou 4, dans laquelle lesdites entités stabilisantes sont fournies sous la forme d'une solution métallo-organique.

6. Composition selon l'une quelconque des revendications précédentes, dans laquelle lesdites entités stabilisantes sont des entités de silicone.

7. Composition selon la revendication 6, dans laquelle lesdites entités de silicone sont de l'orthosilicate de tétrapropyle.

8. Procédé en vue de fabriquer une composition frittée artificielle stabilisée de phases de phosphate de calcium ayant une morphologie appropriée pour le soutien de l'activité cellulaire osseuse sur celles-ci, ledit procédé comprenant les étapes consistant à :
- additionner à une substance d'hydroxyapatite des entités stabilisantes sélectionnées parmi le groupe constitué des entités de silicone, des entités d'aluminium, des entités de zirconium, des entités de baryum, des entités de titane, des entités de germanium, des entités de chrome, des entités de vanadium, des entités de niobium, des entités de bore et de mélanges de celles-ci ;
- fritter ladite substance d'hydroxyapatite additionnée,
dans lequel le frittage convertit ladite substance d'hydroxyapatite additionnée principalement en du phosphate d'alpha-tricalcium au sein de phases de phosphate, dans lequel ledit phosphate d'alpha-tricalcium stabilisé est insoluble dans les fluides physiologiques d'un pH d'environ 6,4 à 7,3, est résorbable par des ostéoclastes et promeut la sécrétion de matrice collagène minéralisante par des ostéoblastes, le procédé étant **caractérisée en ce que** ladite substance d'hydroxyapatite est sous la forme d'un sol-gel et **en ce que** ledit sol-gel d'hydroxyapatite est additionné uniformément desdites entités stabilisantes.

9. Procédé selon la revendication 8, dans laquelle lesdites entités stabilisantes sont sous la forme d'une solution métallo-organique.

10. Procédé selon la revendication 9, dans laquelle l'étape d'addition comprend la dispersion desdites entités stabilisantes dans l'ensemble dudit sol-gel d'une manière qui entoure les surfaces extérieures desdites phases de phosphate.

11. Procédé selon la revendication 8, 9 ou 10, dans laquelle le sol-gel d'hydroxyapatite est fabriqué en mélangeant une solution de tétrahydrate de nitrate de calcium et une solution d'orthophosphate de dihydrogène d'ammonium.

12. Procédé selon la revendication 8, 9, 10 ou 11, dans lequel la composition formée est une poudre, un revêtement ou un matériau brut tridimensionnel.

13. Procédé selon la revendication 8, 9, 10, 11 ou 12, dans lequel lesdites entités de silicone sont de l'orthosilicate de tétrapropyle.

14. Procédé selon la revendication 13, dans lequel ledit orthosilicate de tétrapropyle est en solution avec du 2-méthoxyéthanol.

15. Procédé selon l'une quelconque des revendications 8 à 14, dans lequel lesdites phases de phosphate de calcium sont dans un rapport de 50:50 à 20:80 pour le rapport d'hydroxyapatite sur phosphate d'alpha-tricalcium.

16. Procédé selon l'une quelconque des revendications 8 à 15, dans lequel le frittage de la substance d'hydroxyapatite est effectué à des températures comprises entre environ 900°C et 1100°C.

17. Structure polycristalline microporeuse artificielle frittée en vue du soutien de l'activité cellulaire osseuse, ladite structure étant fabriquée par le procédé selon l'une quelconque des revendications 8 à 15.

18. Structure polycristalline selon la revendication 17, dans laquelle ladite structure est un implant.

19. Méthode in vitro en vue de la mise en culture de cellules osseuses fonctionnelles, ladite méthode comprenant l'application d'une suspension de cellules osseuses dans des milieux physiologiques à une composition selon l'une quelconque des revendications 1 à 7 fournie sur un substrat.
